# NEUE EUROPÄISCHE PATENTSCHRIFT

(11) **EP 1 651 290 B2**
(45) Veröffentlichungstag und Bekanntmachung des Hinweises auf die Entscheidung über den Einspruch: **30.05.2018**
(45) Hinweis auf die Patenterteilung: 18.01.2012
(21) Anmeldenummer: 04763480.3
(22) Anmeldetag: 24.07.2004
(51) Int. Cl.: A61M 1/10

(54) **INTRAKARDIALE PUMPVORRICHTUNG**
INTRACARDIAC PUMP DEVICE
DISPOSITIF DE POMPE INTRACARDIAQUE

(30) Priorität: 08.08.2003 DE 10336902
(43) Veröffentlichungstag der Anmeldung: 03.05.2006
(73) Patentinhaber: Abiomed Europe GmbH, 52074 Aachen (DE)
(72) Erfinder: SIESS, Thorsten, 52146 Würselen (DE)
(74) Vertreter: Cohausz & Florack
(86) Internationale Anmeldenummer: PCT/EP2004/008321
(87) Internationale Veröffentlichungsnummer: WO 2005/016416

(56) Entgegenhaltungen:
- EP-A- 0 445 782
- EP-A- 0 916 359
- WO-A-01/83016
- FR-A- 2 788 223
- US-A- 5 061 256
- US-A1- 2001 000 528

## Beschreibung

Die Erfindung betrifft eine intrakardiale Pumpvorrichtung, die vollständig in das Herz über angrenzende Gefäße eingeführt werden kann, um die natürliche Pumpfunktion des Herzens zu unterstützen oder durch kontinuierlichen Pumpbetrieb zu ersetzen.

Intrakardiale Blutpumpen, die perkutan in den Patientenkörper eingeführt werden, sind stark miniaturisiert. Sie weisen einen zylindrischen Antriebsteil und einen zylindrischen Pumpenteil auf. Das Ansaugende des Pumpenteils ist mit einer flexiblen Kanüle versehen, die am distalen Ende einen Saugkopf mit seitlichen Einlassöffnungen aufweist. Eine solche Pumpvorrichtung ist in EP 0 916 359 A1 (Impella) beschrieben. Eine andere Pumpvorrichtung, die in distaler Richtung fördert, ist in WO 99/58170 (Impella) beschrieben. Bei dieser Pumpvorrichtung ist der Pumpenteil mit einer flexiblen Kanüle verlängert, die durch eine Herzklappe hindurchgeführt werden kann. Aus dem distalen Ende der Kanüle ragt ein Katheter heraus, an dem sich ein Ballon befindet, welcher beim Einführen der Pumpvorrichtung in dem Körper vom Blutstrom mitgenommen werden soll.

Eine Pumpvorrichtung, die das Blut durch eine Kanüle hindurch ansaugt und dann in proximaler Richtung fördert, kann so verlegt werden, dass sie durch die Aortenklappe hindurchführt, wobei der Saugkopf am Ende der Kanüle sich in der linken Herzkammer befindet, während der Pumpenauslass in der Aorta liegt. Die Tätigkeit der kontinuierlich fördernden Pumpe ist der pulsierenden Tätigkeit des Herzens überlagert, so dass die Pumpe starken pulsierenden Druckschwankungen ausgesetzt ist. Dabei kommt es vor, dass die Pumpe zusammen mit dem zugehörigen proximalen Katheter erheblichen Positionsänderungen ausgesetzt ist. Während einer Systole wird der Katheter gegen die Außenseite des Aortenbogens gedrückt und während einer Diastole gegen die Innenseite. Außerdem ändert sich die Lage der Pumpe ständig, wobei es zu Verschiebungen der durch die Aortenklappe hindurchgehenden Kanüle kommen kann, bis hin zu einem Auswurf der Kanüle, die dann aus der Herzklappe in die Aorta entgleitet.

Eine andere Schwierigkeit bei derartigen Blutpumpen besteht darin, dass der Saugkopf der Kanüle sich an Gewebeteilen im Innern des Herzens festsaugen kann. Dadurch besteht die Gefahr von Irritationen des Herzens und ferner wird die Pumpenleistung durch Verstopfung von Einlassöffnungen verringert. Schließlich kann es vorkommen, dass die Kanüle sich an der Mitralklappe festsaugt und durch Ansaugen eine zusätzliche Blutschädigung induziert wird.

Eine Pumpvorrichtung, von der der Oberbegriff des Patentanspruchs 1 ausgeht, ist beschrieben, in U.S. 5,061,256 A. Diese Pumpvorrichtung weist eine Pumpe auf, deren Saugseite mit einer flexiblen Kanüle verbunden ist. Am distalen Ende der Kanüle befindet sich eine schräge Einlassöffnung und proximal von dieser sind an der Kanüle mehrere Hilfsöffnungen vorgesehen. Die größere Einlassöffnung bildet das distale Kanülenende. Die Hilfsöffnungen dienen dazu, ein Kollabieren der Kanülenspitze durch Ansaugen zu vermeiden. Sie verhindern jedoch nicht generell das Ansaugen der größeren Einlassöffnung an Wandteilen des Herzens.

Der Erfindung liegt die Aufgabe zugrunde, eine intrakardiale Pumpvorrichtung zur perkutanen Einführung zu schaffen, bei der die Gefahr des Festsaugens weitgehend vermieden wird.

Die Pumpvorrichtung nach der Erfindung weist die Merkmale des Patentanspruchs 1 auf. Hiernach ist an der Kanüle distal von den Einlassöffnungen ein flexibler Fortsatz vorgesehen. Der Fortsatz bildet ein mechanisches Distanzteil, das einen Abstand zu benachbarten Wänden hält, die Pumpvorrichtung hydraulisch jedoch nicht verändert.

Außer der Distanzhaltefunktion hat der Fortsatz noch weitere Wirkungen. Er vergrößert die mechanische Länge der Pumpvorrichtung, ohne die hydraulische Länge zu vergrößern. Die Vergrößerung der mechanischen Länge hat zur Folge, dass die Pumpvorrichtung weniger leicht durch die Aortenklappe entgleitet. Andererseits wird der hydraulische Widerstand der Kanüle nicht vergrößert, so dass die Saugleistung nicht beeinträchtigt wird. Eine weitere Wirkung besteht darin, dass durch den Fortsatz die Neigung zu pulsierenden Bewegungen der Pumpvorrichtung aufgrund der Herzpulsation erheblich vermindert wird. Die Pumpvorrichtung einschließlich der Pumpe und des Katheters liegt im Herzen wesentlich ruhiger, wodurch auch die Gefahr eines Auswerfens vermindert ist. Für den Fall, dass nach einem Auswerfen ein erneutes Einführen ermöglicht werden soll, ist der distale Fortsatz vorzugsweise derart gestaltet, dass eine erneute retrograde Passage der Aortenklappe leicht und reproduzierbar möglich ist.

Gemäß einer bevorzugten Ausgestaltung der Erfindung ist der flexible Fortsatz ein hohler Schlauch, dessen Lumen mit demjenigen der Kanüle in Verbindung steht. Eine derartige Pumpvorrichtung ist dazu geeignet, mit einem Führungsdraht verwendet zu werden. Der Führungsdraht kann beim Einführen der Pumpvorrichtung als Versteifung eingelegt sein. Es ist auch möglich, zunächst den Führungsdraht zu verlegen und dann die Pumpvorrichtung darüber zu schieben. Schließlich kann auch der Führungsdraht mit einer abgebogenen Spitze aus dem Fortsatz heraus vorgeschoben werden, um als Wegfinder durch das Gefäßsystem zu dienen. Obwohl das Lumen des Fortsatzes mit dem Kanülenlumen in Verbindung steht, saugt die Pumpe nicht über den Fortsatz an. Dies liegt daran, dass die Einlassöffnungen am Saugkopf einen viel größeren Querschnitt haben als das Lumen des Fortsatzes, so dass das Ansaugen wegen des geringeren Strömungswiderstandes weit überwiegend durch die Einlassöffnungen erfolgt. Eine gewisse Saugwirkung, die das Lumen des Fortsatzes ausübt, ist so klein, dass sie vernachlässigbar ist und nicht ausreicht, um ein Festsaugen an anderen Teilen zu bewirken. Der Fortsatz hat daher - anders als der Saugkopf - nicht die Fähigkeit, sich festzusaugen. Wird jedoch durch irgendwelche Umstände das Lumen des Fortsatzes verstopft, so hat dies keine Auswirkungen auf die hydraulische Funktion der Pumpe.

Der flexible Fortsatz weist eine Pigtail-Spitze auf, wie sie von Kathetern und Stents bekannt ist. Die gerundete Pigtail-Spitze ermöglicht ein atraumatisches Abstützen an Herz- oder Gefäßwänden. Im Übrigen ist die Spitze so weich und flexibel, dass sie sich durch Deformation jeglicher Hohlraumtopologie anpasst. Die Pigtail-Spitze erleichtert auch das Einführen und Verlegen der Pumpvorrichtung. Insbesondere kann sie in Verbindung mit einem Führungsdraht benutzt werden, wobei während des Einführens die Pigtail-Spitze durch den Führungsdraht gestreckt wird. Wird die Pigtail-Spitze ohne Führungsdraht vorgeschoben, so ist dennoch eine einfache und reproduzierbare retrograde Passage der Aortenklappe möglich. Dieses ist von besonderer Bedeutung, da für den Betrieb der Pumpe der Führungsdraht entfernt wird und nicht erneut vorgeschoben werden kann, ohne hierfür die Pumpe zu entfernen. Sollte nun die Pumpe infolge der systolischen Herzfunktion aus dem linken Herzen ausgeworfen werden, kann sie aufgrund der Ausformulierung des flexiblen Fortsatzes als Pigtail auf ohne Draht wieder repositioniert werden.

Im Folgenden werden unter Bezugnahme auf die Zeichnungen Ausführungsbeispiele der Erfindung näher erläutert. Die im Zusammenhang mit den Ausführungbeispielen beschriebenen Merkmale schränken den Schutzbereich der Erfindung nicht ein. Dieser wird durch die Patentansprüche bestimmt.

Es zeigen:
- Fig. 1: eine Darstellung der Pumpvorrichtung bei ihrer Funktion innerhalb des Herzens,
- Fig. 2: eine Darstellung der Pumpvorrichtung im unbenutzten Zustand,
- Fig. 3: einen Querschnitt durch den Saugkopf mit daran befestigtem Fortsatz,
- Fig. 4: ein Ausführungsbeispiel, bei dem der Saugkopf einen strömungsformenden Einlauftrichter aufweist, und
- Fig. 5: ein Ausführungsbeispiel, bei dem die Kanüle eine zweite Gruppe von Einlassöffnungen am Ende des Fortsatzes aufweist.

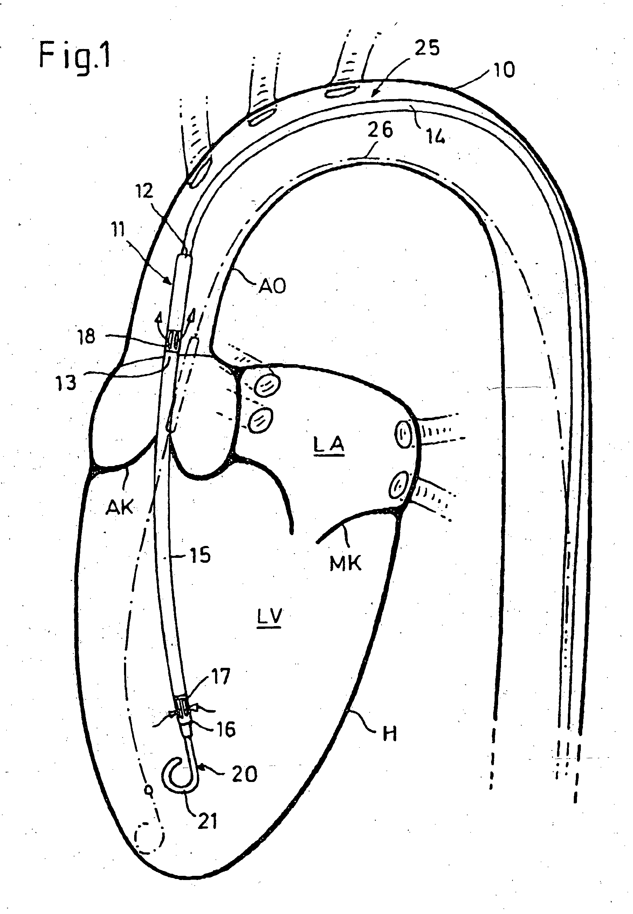

In Figur 1 ist das Herz H mit der davon abgehenden Aorta AO dargestellt. Die Aorta geht über den Aortenbogen 10 in einen vertikalen Strang über, der sich u.a. zur Femoralarterie verzweigt. In die Femoralarterie in der Leistengegend wird die Pumpvorrichtung perkutan eingebracht und bis in das Herz vorgeschoben.

Die intrakardiale Pumpvorrichtung weist eine Pumpe 11 auf, die ein proximales Ende 12 und ein distales Ende 13 hat. Die Pumpe 11 weist ein Gehäuse mit einem Außendurchmesser von maximal 4 mm und einer Länge von etwa 15 mm auf, so dass die Pumpe perkutan eingeführt und intrakardial betrieben werden kann. Größere Pumpen, die aber nur chirurgisch einführbar sind, dürfen 6 mm Außendurchmesser aufgrund der peripheren Gefäßdurchmesser nicht überschreiten.

Das proximale Ende 12 der Pumpe ist mit einem Katheter 14 verbunden, der die elektrischen Leitungen für den Betrieb und die Steuerung der Pumpe 11 enthält. Das distale Ende 13 ist mit einer Kanüle 15 verbunden, die aus einem langgestreckten flexiblen Schlauch besteht, der an seinem distalen Ende ein Saugkopf 16 mit seitlichen Einlassöffnungen 17 bildet. Die Pumpe 11 saugt Blut über die Einlassöffnungen 17 der Kanüle 15 an und pumpt dieses durch die seitlich an der Pumpe vorgesehenen Auslassöffnungen 18. Pumpe und Kanüle sind generell so gestaltet, wie es in EP 0 916 359 A1 (Impella) beschrieben ist. Die Kanüle 15 besteht aus einem Schlauch mit einer Länge von etwa 40 bis 70 mm, dessen Wand aus einer Drahtwendel gebildet ist, welche mit einem Überzug aus Polyuhrethan versehen ist. Die Kanüle 15 hat eine gewisse Formstabilität, ist jedoch flexibel.

Erfindungsgemäß schließt sich an den Saugkopf 16 der Kanüle 15 ein Fortsatz 20 an, der die Kanüle 15 mechanisch verlängert, nicht aber hydraulisch. Der Fortsatz 20 hat eine Länge von 10 bis 30 mm. Er ist hier mit einer Pigtail-Spitze 21 versehen, um atraumatische Abstützungen an Körpergewebe zu ermöglichen.

In Figur 1 ist in durchgezogenen Linien der Verlauf der Pumpvorrichtung in Herz H und Aorta AO dargestellt. Die Pumpvorrichtung ist so verlegt, dass die Pumpe 11 sich in der Aorta AO befindet, während der Saugkopf 16 im linken Ventrikel LV liegt. Die Kanüle 15 erstreckt sich durch die Aortenklappe AK. Die Pumpe saugt also im linken Ventrikel LV an und fördert in die Aorta AO. Außerdem sind in Figur 1 das linke Atrium LA und die Mitralklappe MK erkennbar.

Die Pumpe 11 pumpt kontinuierlich mit einer Förderleistung von 2 bis 3 l/min. Die Reaktionskraft ist bestrebt, die Pumpe in das Herz hineinzuziehen. Dieser Kraft wirkt die Pumpkraft des Herzens entgegen. Dieses hat fluktuierend während der Systole eine Förderleistung von etwa 10 l/min. Es hat sich herausgestellt, dass die Pumpe Bewegungen ausführt, wobei sich während der Auswurfphase des Herzens eine systolische Lage 25 an der Außenseite des Aortenbogens 10 ergibt und während der Füllphase eine diastolische Lage 26 an der Innenseite des Aortenbogens. Mit diesen Bewegungen ändert sich auch die Lage der Kanüle 15 und des Saugkopfes 16. Wenn der Saugkopf 16 in die Nähe der an der Herzwand befindlichen Trabekelstrukturen kommt, besteht die Gefahr des Ansaugens dieser Strukturen, der Verstopfung des Saugkopfes, der erhöhten Blutschädigung sowie die Gefahr einer Hämatombildung an der kardialen Struktur.

Das Festsaugen wird durch den Fortsatz 20 erschwert, der sich an der Herzwand abstützt. Außerdem bildet der Fortsatz 20 eine mechanische Verlängerung der Kanüle zur Verhinderung des Auswerfens aus dem linken Ventrikel und der Aortenklappe.

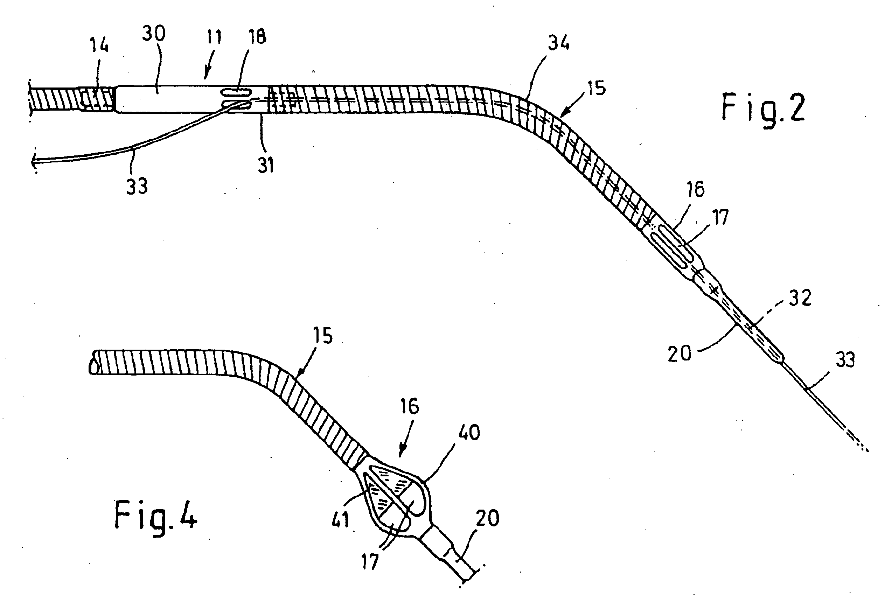

Wie aus Figur 2 hervorgeht, weist die Pumpe 11 einen Motorteil 30 und einen Pumpenteil 31 auf, die axial hintereinander angeordnet sind. Der Pumpenteil 31 enthält einen Gehäusering und ein von dem Motor angetriebenes Flügelrad, das den Blutstrom in axialer Richtung fördert, wobei der Blutstrom radial nach außen abgelenkt wird und durch die Auslassöffnungen 18 seitlich aus dem Gehäuse der Pumpe 11 austritt. An den Pumpenteil 31 schließt sich axial die Kanüle 15 an, die in etwa den gleichen Außendurchmesser (4 mm) hat wie die Pumpe 11. Der Saugkopf 16 mit den Einlassöffnungen 17 hat eine Länge von etwa 10 bis 15 mm. Die Auslassöffnungen 18 haben eine Fläche, die mindestens so groß ist wie die Querschnittsfläche des Kanülenlumens, so dass der Saugkopf keine Verengungsstelle bildet.

Gemäß Figur 2 ist der Fortsatz 20 als hohler Schlauch ausgebildet, der ein durchgehendes Lumen 32 hat. Die Weite dieses Lumens ist viel kleiner als diejenige des Kanülenlumens. Das Lumen 32 dient zum Hindurchstecken eines Führungsdrahtes 33 zur Erleichterung des Einführens der Pumpvorrichtung in den Körper. Der Führungsdraht 33 streckt den Fortsatz 20, wenn dieser eine vorgeformte Krümmung aufweist. Der Führungsdraht kann auch eine weichflexible gebogene Spitze aufweisen, die aus dem distalen Ende des Fortsatzes 20 herausragt und als Wegfinder durch das Gefäßsystem dient. Der Führungsdraht 33 führt in die Pumpe 11 hinein, durch den Pumpenteil 31 hindurch und aus einer Auslassöffnung 18 hinaus. Er wird dann außen an dem Katheter 14 entlanggeführt. Nach dem Verlegen der Pumpvorrichtung wird der Führungsdraht herausgezogen.

Wie aus Figur 2 hervorgeht, weist die Kanüle 15 eine Vorbiegung 34 auf, die ebenfalls der besseren Wegfindung dient.

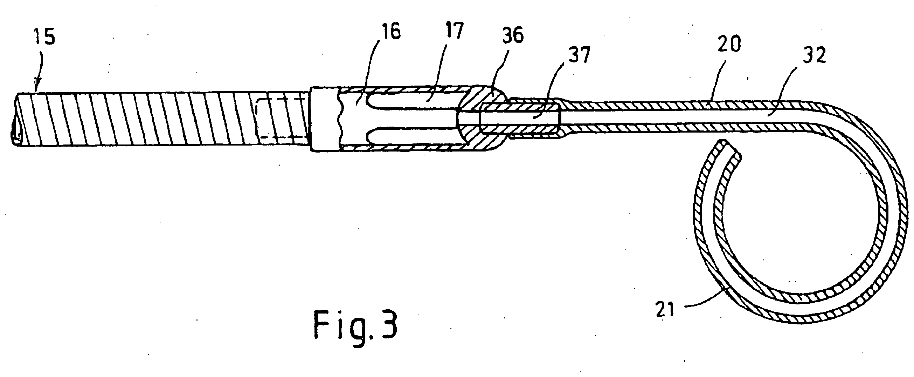

In Figur 3 ist das distale Ende der Kanüle 15 mit dem Saugkopf 16 dargestellt. Der Saugkopf 16 weist die länglichen Einlassöffnungen 17 auf. An seinem Ende befindet sich eine Kugel 36, in die ein hohler Stift 37 eingesetzt und eingeschweißt ist. Der Stift 37 dient als Verbindungselement für den Fortsatz 20, der mit einer Pigtail-Spitze 21 versehen ist. Das Lumen 32 des Fortsatzes 20 erstreckt sich durch den Stift 37 und die Kugel 36 hindurch in den Saugkopf 16 hinein. Der Außendurchmesser des Fortsatzes 20 ist hier kleiner als derjenige der Kanüle 15.

Figur 4 zeigt ein Ausführungsbeispiel, bei dem der Saugkopf 16 in einem expandierbaren Ansaugkorb 40 einen Einlauftrichter 41 enthält. Der Ansaugkorb 40 ist beispielsweise aus einem rückstellfähigen Material hergestellt oder er wird durch einen Ballon aufgeweitet. Im aufgeweiteten Zustand hat der Ansaugkorb 40 einen Außendurchmesser, der größer ist als derjenige der Kanüle 15. So wird der Ansaugkopf von ursprünglich 4 mm auf einen Durchmesser von ca. 6 mm aufgeweitet. Dabei wird der Einlauftrichter 41 aus einem flexiblen Polymerschirm aufgespannt, der eine glatte Einströmung ermöglicht und durch Verringerung der hydraulischen Verluste die hydraulische Leistung der Pumpe wesentlich erhöht.

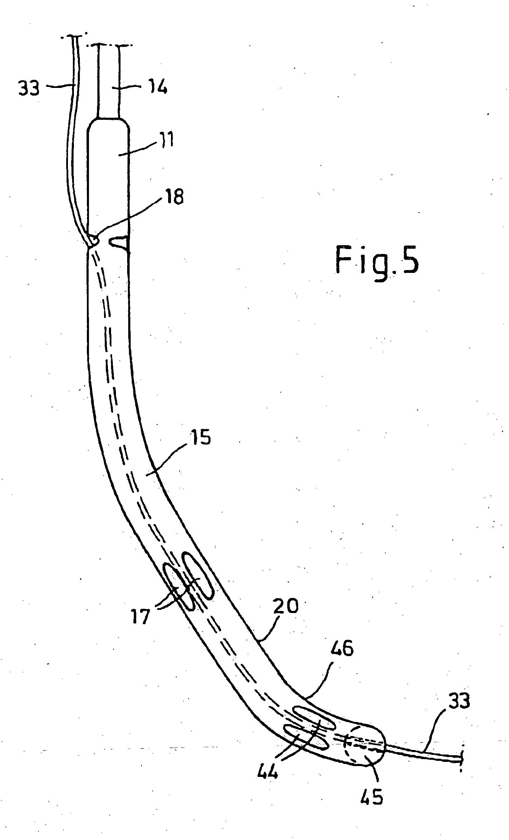

Bei dem Ausführungsbeispiel nach Figur 5 ist die Kanüle 15 zweistufig ausgebildet. Sie ist mit ersten Einlassöffnungen 17 versehen, die den Haupteinlass bilden. Distal von den Einlassöffnungen 17 schließt sich der Fortsatz 20 an, der in diesem Fall den gleichen Außendurchmesser und den gleichen Lumendurchmesser hat, wie die Kanüle 15. Am distalen Endbereich des Fortsatzes 20 befinden sich weitere seitliche Zusatzöffnungen 44, die als Zusatzöffnungen dienen. Ein Saugkopf ist hierbei nicht vorhanden. Das distale Ende der Kanüle ist mit einer abgerundeten Stirnwand 45 verschlossen, die einen Durchlass für einen Führungsdraht 33 aufweist.

Die Pumpvorrichtung nach Figur 5 wird über den Führungsdraht 33 aufgeschoben, der durch den Impeller-Teil der Pumpe 11 hindurchgeht und seitlich aus einer Auslassöffnung 18 austritt. Nach der Positionierung der Pumpvorrichtung wird der Führungsdraht 33 in proximaler Richtung herausgezogen. Die Funktion der Pumpe 11 bewirkt ein Ansaugen von Blut durch die Einlassöffnungen 17. Infolge des höheren Strömungswiderstandes des Fortsatzes 20 und der Zusatzöffnungen 44 üben nur die Einlassöffnungen 17 eine Ansaugwirkung aus, während der Ansatz 20 hydraulisch praktisch unwirksam bleibt. Nur wenn die Einlassöffnungen 17 sich festsaugen oder auf andere Weise verstopft werden, treten die Zusatzöffnungen 44 in Funktion. Der Fortsatz 20 weist eine Abwinklung 46 auf. Sein Lumen ist mit demjenigen der Kanüle 15 verbunden. Der Fortsatz hat eine Flexibilität, die vorzugsweise größer ist als diejenige der Kanüle 15.

## Patentansprüche

1. Intrakardiale Pumpvorrichtung zur perkutanen Einführung, mit einer Pumpe (11), die am proximalen Ende (12) mit einem Katheter (14) und am saugseitigen distalen Ende (13) mit einer Kanüle (15) verbunden ist, welche entfernt von der Pumpe Einlassöffnungen (17) aufweist, wobei an der Kanüle distal von den Einlässöffnungen (17) ein flexibler Fortsatz (20) vorgesehen ist, **dadurch gekennzeichnet, dass** der flexible-Fortsatz (20) eine Pigtail-Spitze (21) aufweist und dass die Einlassöffnungen (17) an einem expandierenden Ansaugkorb (40) vorgesehen sind, der einen Einlauftrichter (41) enthält.

2. Pumpvorrichtung nach Anspruch 1, **dadurch gekennzeichnet, dass** der Fortsatz (20) ein nichtsaugender Fortsatz ist.

3. Pumpvorrichtung nach Anspruch 1 oder 2, **dadurch gekennzeichnet, dass** der Fortsatz (20) ein hohler Schlauch ist, dessen Lumen mit demjenigen der Kanüle (15) in Verbindung steht.

4. Pumpvorrichtung nach einem der Ansprüche 1 - 3, **dadurch gekennzeichnet, dass** der Fortsatz (20) einen Außendurchmesser hat, der kleiner ist als derjenige der Kanüle (15).

5. Pumpvorrichtung nach einem der Ansprüche 1 - 4, **dadurch gekennzeichnet, dass** die Kanüle (15) eine Vorbiegung (34) aufweist.

6. Pumpvorrichtung nach einem der Ansprüche 1 - 5, **dadurch gekennzeichnet, dass** ein Führungsdraht (33) vorgesehen ist, der durch die Pumpe (11) hindurchführt und aus der Kanüle (15) in den hohlen Fortsatz (20) vorschiebbar ist.

7. Pumpvorrichtung nach einem der Ansprüche 1 und 3 - 6, **dadurch gekennzeichnet, dass** der Fortsatz (20) seitliche Zusatzöffnungen (44) aufweist.

## Claims

1. An intracardiac pumping device for percutaneous insertion, comprising a pump (11) connected at the proximal end (12) with a catheter (14) and at the suction-side distal end (13) with a canula (15) having inlet openings (17) remote from the pump, wherein a flexible projection (20) is provided at the canula distal of the inlet openings (17), **characterized in**
**that** the flexible projection (20) has a pigtail tip (21) and
**that** the inlet openings (17) are provided in an expansible suction basket (40) including an inflow funnel (41).

2. The pumping device of claim 1, **characterized in that** the projection (20) is a non-sucking projection.

3. The pumping device of claim 1 or 2, **characterized in that** the projection (20) is a hollow hose whose lumen is in communication with that of the canula (15).

4. The pumping device of one of claims 1-3, **characterized in that** the projection (20) has an outer diameter that is smaller than that of the canula (15).

5. The pumping device of one of claims 1-4, **characterized in that** the canula (15) has a preformed bend (34).

6. The pumping device of one of claims 1-5, **characterized in that** a guide wire (33) is provided that leads through the pump (11) and is adapted to be advanced from the canula (15) into the hollow projection (20).

7. The pumping device of one of claims 1 and 3-6, **characterized in that** the projection (20) has lateral auxiliary openings (44).

## Revendications

1. Dispositif de pompe intracardiaque destiné à l'insertion percutanée, comprenant une pompe (11) connectée à son extrémité proximale (12) avec un cathéter (14) et connectée à son extrémité (13) distale côté aspiration avec une canule (15) qui a des ouvertures d'entrée (17) loin de la pompe, une saillie (20) flexible étant prévue à ladite canule à une position distale des ouvertures d'entrée (17),
**caractérisé en ce que**
ladite saillie (20) flexible comprend une pointe du type pigtail (21) et lesdites ouvertures d'entrée (17) sont prévues dans une corbeille d'aspiration (40) expansible qui comprend une trémie d'entrée (41).

2. Dispositif de pompe selon la revendication 1, **caractérisé en ce que** ladite saillie (20) est une saillie non-aspiratoire.

3. Dispositif de pompe selon les revendications 1 ou 2, **caractérisé en ce que** ladite saillie (20) est un tuyau creux dont la lumière est en communication avec celle de ladite canule (15).

4. Dispositif de pompe selon les revendications 1 - 3, **caractérisé en ce que** ladite saillie (20) a un diamètre extérieur qui est inférieur à celui de la canule (15).

5. Dispositif de pompe selon les revendications 1 - 4, **caractérisé en ce que** la canule (15) a une courbure préformée (34).

6. Dispositif de pompe selon les revendications 1 - 5, **caractérisé en ce qu'**un fil de guidage (33) est prévu qui s'étend à travers ladite pompe (11) et est apte à être avancé hors de la canule (15) dans ladite saillie (20) creuse.

7. Dispositif de pompe selon les revendications 1 et 3 - 6, **caractérisé en ce que** ladite saillie (20) comprend des ouvertures (44) latérales auxiliaires.
